# EUROPEAN PATENT SPECIFICATION

(11) **EP 1 608 784 B1**
(45) Date of publication and mention of the grant of the patent: **14.01.2009**
(21) Application number: 04757932.1
(22) Date of filing: 19.03.2004
(51) Int. Cl.: C12Q 1/68

(54) **GLOBAL LINEAR NON-BIASED NUCLEIC ACID AMPLIFICATION**
METHODE ZUR LINEAREN NICHT-SELEKTIVEN AMPLIFIKATION VON NUKLEINSÄUREN
AMPLIFICATION GLOBALE LINEAIRE NON BIAISEE D'ACIDE NUCLEIQUE

(30) Priority: 21.03.2003 US 456825 P
(43) Date of publication of application: 28.12.2005
(73) Proprietor: MDS Analytical Technologies (US) Inc., Sunnyvale, CA 94089 (US)
(72) Inventor: ERLANDER, Mark, G., Encinitas, California 92024 (US); SALUNGA, Ranelle, C., San Digeo, CA 92111 (US); MA, Xiao-Jun, San Diego, CA 92130 (US); ENRIGHT, Eddy, San Diego, CA 92130 (US)
(74) Representative: Roques, Sarah Elizabeth
(86) International application number: PCT/US2004/008553
(87) International publication number: WO 2004/085681

(56) References cited:
- WO-A-90/01065
- WO-A-99/25873
- WO-A-02/052031
- BAUGH L R ET AL: "Quantitative analysis of mRNA amplification by in vitro transcription" NUCLEIC ACIDS RESEARCH, OXFORD UNIVERSITY PRESS, SURREY, GB, vol. 29, no. 5, 1 March 2001 (2001-03-01), pages 1-9, XP002301337 ISSN: 0305-1048

## Description

### RELATED APPLICATIONS

This application claims benefit of priority from provisional U.S. Patent Application 60/456,825, filed March 21, 2003, which is hereby incorporated in its entirety as if fully set forth.

### TECHNICAL FIELD OF THE INVENTION

The technical field of this invention is enzymatic amplification of nucleic acids. More particularly, the invention provides methods, compositions and kits relating to amplifying (i.e., making multiple copies of) target polynucleotides to produce multiple copies thereof. The multiple copies may contain either the sense or antisense sequence of the amplified target polynucleotide. The invention also provides amplification of target polynucleotides, even if present in limited quantities, for use in subsequent analytical or preparative purposes.

### BACKGROUND

Differential expression analysis of mRNA species in a test population requires the quantitative determination of different mRNA levels in the population. Although detection of a nucleic acid and its sequence analysis can be carried out by probe hybridization, the method generally lacks sensitivity when amounts of target nucleic acid in the test sample are low. Low copy number nucleic acid targets are difficult to detect even when using highly sensitive reporter groups like enzymes, fluorophores and radioisotopes. Detection of rare mRNA species is also complicated by factors such as heterogeneous cell populations, paucity of material, and the limits of detection of the assay method. Methods which amplify heterogeneous populations of mRNA also raise concerns with introduction of significant changes in the relative amounts of different mRNA species.

Applications of nucleic acid amplification method include detection of rare cells, pathogens, altered gene expression in malignancy, and the like. Nucleic acid amplification is potentially useful for both qualitative analysis, such as the detection of nucleic acids present in low levels, as well as the quantification of expressed genes. The latter is particularly useful for assessment of pathogenic sequences as well as for the determination of gene multiplication or deletion associated with malignant cell transformation. A number of methods for the amplification of nucleic acids have been described, e.g., exponential amplification, linked linear amplification, ligation-based amplification, and transcription-based amplification. An example of exponential nucleic acid amplification method is polymerase chain reaction (PCR) which has been disclosed in numerous publications. (*see* Mullis et al. Cold Spring Harbor Symp. Quant. Biol. 51:263-273 (1986); PCR Cloning Protocols: From Molecular Cloning to Genetic Engineering, Methods in Molecular Biology, White, B. A., ed., vol. 67 (1998); Mullis EP 201,184; Mullis et al., U.S. Pat. Nos. 4,582,788 and 4,683,195; Erlich et al., EP 50,424, EP 84,796, EP 258,017, EP 237,362; and Saiki R. et al., U.S. Pat. No. 4,683,194). Linked linear amplification is disclosed by Wallace et al. in U.S. Pat. No. 6,027,923. Examples of ligation-based amplification are the ligation amplification reaction (LAR), disclosed by Wu et al. in Genomics 4:560 (1989) and the ligase chain reaction, disclosed in EP Application No. 0320308 B1. Hampson et al. (Nuc1. Acids Res. 24(23):4832-4835, 1996) describe a directional random oligonucleotide primed (DROP) method for use as part of global PCR amplification.

Isothermal target amplification methods include transcription-based amplification methods, in which an RNA polymerase promoter sequence is incorporated into primer extension products at an early stage of the amplification (WO 89/01050), and a target sequence or its complement is amplified by transcription and digestion of the RNA strand in a DNA/RNA hybrid intermediate. (See, for example, U.S. Pat. Nos. 5,169,766 and 4,786,600). These methods include transcription mediated amplification (TMA), self-sustained sequence replication (3SR), Nucleic Acid Sequence Based Amplification (NASBA), and variations thereof. (*See* Guatelli et al. Proc. Natl. Acad. Sci. U.S.A. 87:1874-1878 (1990); U.S. Patent Nos. 5,766,849 (TMA); and 5,654,142 (NASBA)).

Some transcription-based amplification methods (Malek et al., U.S. Pat. No. 5,130,238; Kacian and Fultz, U.S. Pat. No. 5,399,491; Burg et al., U.S. Pat. No. 5,437,990) use primer-dependent nucleic acid synthesis to generate a DNA or RNA, product, which serves as a template for additional rounds of primer-dependent nucleic acid synthesis. These methods use at least two primers each having sequences complementary to different strands of a target nucleic acid sequence and results in an exponential amplification of the number of copies of the target sequence. However, these methods are not amenable for global gene expression monitoring applications.

Amplification methods that utilize a single primer are also useful for amplification of heterogeneous mRNA populations. Since the vast majority of mRNAs comprise a homopolymer of 20-250 adenosine residues on their 3' ends (the poly-A tail), poly-dT primers can be used for cDNA synthesis. "Single-primer amplification" protocols utilize a single primer containing an RNA polymerase promoter sequence and a sequence, such as oligo-dT, complementary to the 3'-end of the desired nucleic acid target sequence(s) ("promoter-primer"). (Kacian et al., U.S. Pat. No. 5,554,516; van Gelder et al., U.S. Pat. Nos. 5,545,522 ('522), 5,716,785 ('785) and 5,891,636 ('636)). These methods use, or could be adapted to use, a primer containing poly-dT for amplification of heterogeneous mRNA populations. In methods described in '522, '785 and '636, the promoter-primer is used to prime the synthesis of a first strand and an endogenously derived primer is used for second strand synthesis. The double-stranded cDNA thus generated includes a promoter coupled to a sequence corresponding to the target RNA and is used as a template for the synthesis of multiple copies of RNA complementary to the target sequence(s) ("antisense RNA") by use of RNA polymerase. The method described in U.S. Pat. No. 5,716,785 has been used to amplify cellular mRNA for monitoring gene expression (e.g., van Gelder et al. (1990), Proc. Natl. Acad. Sci. USA 87, 1663; Lockhart et al. (1996), Nature Biotechnol. 14, 1675).

Another method to produce "antisense RNA" with an RNA polymerase is disclosed by Loewy (U.S. Pat. No. 5,914,229) where a single-stranded nucleic acid of interest is combined with an oligonucleotide containing a double stranded promoter and a single stranded segment complementary to the nucleic acid of interest. Eberwine (BioTechniques 20:584-591 (1996)) disclose yet another means to amplify mRNA and produce "antisense RNA" by using immobilized oligo(dT)-T7 primers to produce the necessary cDNA. Wang et al. (U.S. Pat. no. 5,932,541) disclose the use of a "captureable" primer to produce the first strand of a cDNA before it is immobilized on a solid support (via the "capturable primer) prior to the synthesis of the second cDNA strand.

Another *in vitro* transcription protocol is disclosed by Hughes et al. (Nature Biotech. 19:342-347, April 2001), where a two primer system (modified from US patent 6,132,997) and an adapted PCR coupled system are used.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

### SUMMARY OF THE INVENTION

The present invention provides methods, compositions and kits relating to amplifying target polynucleotides and generating amplified RNA (aRNA). The aRNA generally contains sequences spanning the entire length of the target polynucleotide being amplified. The invention may be practiced with DNA molecules as the target polynucleotides but is preferably practiced with RNA molecules as the target polynucleotides. Thus in one aspect, the invention relates to a general method for amplifying the entire lengths of a plurality of RNA molecules, including a mixture of transfer, ribosomal, and/or messenger RNA molecules (hereafter tRNA, rRNA, and mRNA molecules, respectively).

Preferably, the total RNA species (e.g. tRNA, rRNA and mRNA) in a sample is randomly-primed to produce aRNA molecules ranging from 200-1000 bases. These aRNA molecules may contain fragments of the sequences present in the RNA species that are amplified. Addition, the invention provides for methods that enrich for the amplification of specific species of RNA molecules, especially preferential amplification of mRNA molecules.

The methods of the invention are not limited to amplifying the most 3' end of RNA molecules but rather amplifies overlapping fragments that represent the entire length of all RNA molecules being amplified. Particularly, the invention provides for the enrichment of amplified fragments of mRNAs.

The present invention may be used to amplify the population of RNAs extracted from formalin-fixed tissues and/or the population(s) of mRNA splice variants. The aRNA produced after either of these uses may be analyzed by any means, including hybridization to a microarray of known sequences where the aRNA is labeled as described herein.

The aRNA produced by the practice of the invention may be in the form of either a "sense" RNA molecule containing all or part of the sequence found in the target polynucleotide, or an "antisense" RNA molecule containing a sequence complementary to all or part of the sequence found in the target polynucleotide.

In one aspect of the invention, a double stranded DNA molecule is produced to contain all or part of the sequence of the target polynucleotide of interest as well as at least one promoter capable of initiating the transcription of either strand of the double stranded DNA. The production of the double stranded DNA begins with the initial production of a first strand, "antisense" DNA by hybridizing a strand of the target polynucleotide with a first oligonucleotide comprising a random sequence as a primer. The random sequence may be of various lengths, such that hybridization may occur at various sequences along the length of the target polynucleotide. In one alternate embodiment, the first oligonucleotide is used in combination with an oligo dT primer.

The first oligonucleotide may optionally be directly or indirectly linked, via its 5' end, to a defined (or known) sequence. Similarly, an oligo dT primer used in the practice of the invention may also comprise a defined sequence linked to its 5' end. Preferably, the defined sequence is relatively unhybridizable, or non-complementary according to basepairing rules, to the polynucleotide target.

If the target polynucleotide is single stranded, it may be used directly. If the target polynucleotide is double stranded, it is first denatured to generate a single stranded target polynucleotide. The single stranded target polynucleotide is used as the template for the production of said first strand DNA.

After said hybridizing event, a first strand DNA complementary to the target polynucleotide is produced by extending the first oligonucleotide. Where the target polynucleotide used as the template is a single stranded RNA molecule, enzymatic extension of the first oligonucleotide with reverse transcriptase activity may be used. If the target polynucleotide is DNA, a DNA dependent DNA polymerase activity is used. As an optional embodiment of the invention, excess or residual first oligonucleotides not used to prime first strand DNA molecules are degraded, such as by S 1 nuclease treatment. Alternatively, alkaline phosphatase is added to deplete the reaction of dNTPs.

After production of the first strand DNA, it is extended at its 3' end via terminal transferase activity (such as via use of a terminal deoxyribonucleotyl transferase) to add a 3' homopolymer tail of dA, dG, dT, or dC nucleotides. Preferably, a homopolymer of dT is added. Optionally, the first strand DNA hybrid is first separated from the target polynucleotide template before the tailing reaction with a terminal transferase activity. This may be accomplished by heating the reaction components, which also terminates reverse transcriptase activity.

The first strand DNA is then hybridized to a second oligonucleotide comprising a primer region that is complementary to all or part of the homopolymer tail at the 3' end of said first strand DNA. The second oligonucleotide is directly or indirectly linked, via its 5' end, to a promoter sequence. The promoter sequence can be single stranded such that when it is made double stranded to form a promoter, the promoter is capable of initiating transcription from the promoter and in the direction of the primer sequence that is complementary to the homopolymer tail. Preferably, the promoter sequence is relatively unhybridizable, or non-complementary according to basepairing rules, to the first DNA strand.

After hybridization of the second oligonucleotide, a double stranded DNA is produced by forming a second strand DNA, via primer extension, that is complementary to all or part of the first strand DNA. The primer extension process utilizes a DNA dependent DNA polymerase activity and the necessary deoxyribonucleotides. Preferably, a DNA polymerase with 3' exo activity is used to extend the first DNA strand at its 3' end to be fully complementary to the optional promoter sequence in the second oligonucleotide. This results in a double stranded DNA molecule.

Because the second strand DNA was produced via the use of a second oligonucleotide with a linked promoter sequence, the resultant double stranded DNA has a promoter coupled to the end of the double stranded DNA corresponding to the 3' end of the first strand DNA. The second oligonucleotide is preferably designed to permit the double stranded promoter region to initiate transcription that produces RNA containing all or part of the primer sequence and any optional "linker" sequence present in the second oligonucleotide.

In another aspect of the invention, and after production of the double stranded DNA, the promoter present on the DNA is contacted with RNA polymerase capable of initiating transcription from the promoter to transcribe one or more copies of an amplified RNA (aRNA) complementary to sequences present on the first strand DNA. Transcription initiated from the promoter linked to the second oligonucleotide sequence expresses aRNA comprising in a 5' to 3' order, the optionally present "linker" sequence, the second primer sequence, a sequence that is all or part of the target polynucleotide, and a sequence complementary to the first oligonucleotide. The resultant aRNA would thus be "sense" relative to the target polynucleotide of interest.

The above methods may be viewed as "round one" amplification to produce "sense" aRNA.

In another aspect of the invention, "round two" amplification is provided to enable further amplification of "sense" aRNA. Round two amplification is possible by using the above aRNA to produce multiple copies of double stranded DNA constructs to further amplify the target polynucleotide. In round two, the "sense" aRNA is used to first produce another first strand DNA. A "round two" first strand DNA is produced by first hybridizing the aRNA with one or more first oligonucleotides as described above. The "round two" first strand DNA is produced upon extension of the first oligonucleotide(s) with reverse transcriptase activity, with the "sense" aRNA acting as the template. After production of the "round two" first strand DNA, it is (optionally separated from the aRNA template and) hybridized with a "round one" second oligonucleotide which is complementary to the 3' end of the first strand DNA. Extension of the second oligonucleotide produces a "round two" second strand DNA, which, hybridized to the first strand DNA, forms a double stranded DNA molecule.

Transcription initiated from the promoter linked to the 3' end of the first strand DNA results in production of one or more copies of "round two" aRNA, which contain sequences of the "sense" aRNA used as the starting template in "round two." Preferably, this round two aRNA comprises in a 5' to 3' order, the optionally present "linker" sequence of the second oligonucleotide, the primer sequence of the second oligonucleotide, a sequence that is all or part of the "sense" aRNA (and thus all or part of the original target polynucleotide), and a sequence complementary to the first oligonucleotide(s) used. The resultant aRNA would again be "sense" relative to the original target polynucleotide of interest.

Use of "round two" permits significant further amplification of the target polynucleotide because the quantity of "round one" aRNA is used to prepare multiple "round two" double stranded DNAs which may then be used to produce even larger amounts of aRNA upon transcription.

In an alternative embodiment of "round two" where the first oligonucleotide used in "round one" comprised a defined sequence at its 5' end, the oligonucleotides used to prime synthesis of the first strand DNA in "round two" may optionally contain a promoter sequence directly or indirectly coupled to the 5' end of a primer sequence which is all or part of said defined sequence. Extension of this oligonucleotide, followed by priming and extension with a "round one" second oligonucleotide, results in a double stranded DNA molecule wherein both strands can serve as a template for transcription initiated from the promoter coupled to the "round one" second oligonucleotide and/or the promoter coupled to the oligonucleotide used to prime synthesis of the first strand DNA in "round two". This possible embodiment permits an embodiment wherein the two promoter regions are different. This results in the double stranded DNA being able to produce "sense" aRNA by transcription initiated from the promoter coupled to the "round one" second oligonucleotide and "antisense" aRNA by transcription initiated from the other promoter. The terms "sense" and "antisense" remain relative to the original target polynucleotide.

It should be noted that in all of the above methods, "exogenous primers" are present at least in the form of the oligonucleotides used to prime synthesis of the second DNA strand in "round one" or the DNA strands in "round two."

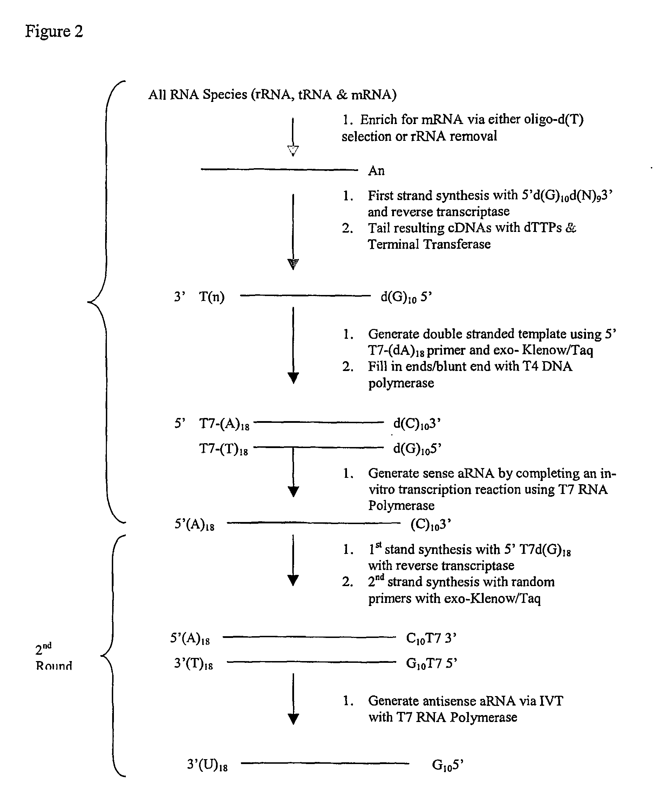

In another alternative embodiment of the invention, "round two" can be used to remove a promoter coupled to the 3' end of the first strand DNA of "round one" in favor of a promoter coupled to the 5' end of the first strand DNA of "round one". This embodiment is shown in Figure 2 and further described below.

The methods of the present invention may be used to detect a DNA or RNA molecule of interest from a cell or organism. Preferably the cell is a eukaryotic or human cell, more preferred are cells from malignant cells, such as those associated with cancer, especially breast cancer. The present methods may be used to amplify a population of RNA molecules obtained (extracted) from a fixed sample, including, but not limited to a formalin fixed and paraffin embedded (FFPE), a formalin fixed, or alcohol fixed sample. The degraded (or modified) nature of the RNA in a given cell/tissue/organism of such a sample does not prevent the methods of the invention from amplifying the RNA in a global manner (such that all RNA molecules may be amplified) and in a non-biased manner (such that no bias for particular RNA molecules or particular regions of RNA molecules results). The utility of the methods of the invention includes any situation in which any or all RNA species of a biological sample or source need to be amplified to generate more RNA. Non-limiting examples of such situations include cases where there is a low amount of RNA available (e.g., < 1 ng of total RNA). In one optional embodiment, the resultant amplified RNA may be used as the template for PCR or quantitative PCR reactions for specific RNA molecules.

In preferred embodiments of the invention, the entire RNA population (including rRNA, tRNA, and mRNA) from one or more than one cell that is laser-captured (laser capture microdissection) from fixed tissues from model organisms of human diseases or actual human tissue (postmortem or biopsy material) is amplified. Alternatively, a particular subpopulation of RNA (e.g. mRNA or rRNA) molecules may be selected for amplification as disclosed below.

More than one cell includes a plurality or other multitude of cells, from a cell culture or a tissue or cell type therein. Cells that may be used in the practice of the present invention include, but are not limited to, primary cells, cultured cells, tumor cells, non-tumor cells, blood cells, cells of the the pituitary or other endocrine glands, bone cells, lymph node cells, brain cells, lung cells, heart cells, spleen cells, liver cells, kidney cells, and vascular tissue cells. Beyond cancer cells, the present invention may be applied to tissues (and cell types therein) involved in, or associated with, any disease or undesired condition. For example, and without limiting the invention, the present invention may be used to determine gene expression in neuronal and non-neuronal cells involved in disorders of the nervous system, such as, but not limited to, neurodegenerative diseases, including Parkinson's disease and Alzheimer's disease; multiple sclerosis; and psychiatric disorders, including schizophrenia and affective disorders such as manic depression, lack of apetitite control, and attention deficit disorder. Expressed nucleic acids from different neuronal cell types involved in or associated with the above disorders, either by single or multiple cells of the same type or subtype, may be amplified with the present invention for further characterization. Similarly, expressed nucleic acids from non-neuronal cells associated with such disorders (including, but not limited to microglial cells, astrocytes, oligodendricytes, and infiltrating inflammatory cells) may also be amplified with the present invention.

Also without limiting the invention, expressed nucleic acids from cells associated with disorders of the cardiovascular and urinary systems may be amplified with the present invention. Examples from the area of cardiovascular disease include, but are not limited to, smooth muscle cells, endothelial cells and macrophages while examples from kidney disorders include, but are not limited to, cells of the cortex, medulla, glomerulus, proximal and distal tubules, Bowman's capsule and the Loop of Henley.

Inflammatory and autoimmune diseases are additional non-limiting examples of disorders wherein the tissues and cells involved in or associated therewith may be used in combination with the present invention. Examples of such disorders include rheumatoid arthritis, myasthenia gravis, lupus erythematosus, certain types of anemia, multiple sclerosis, and juvenile-onset diabetes. Cells involved in such diseases include neutrophils, eosinophils, basophils, monocytes, macrophages, lymphocytes,

Additional examples of cancer cells which may be used in conjunction with the present invention include, but are not limited to, cells from sarcomas, carcinomas, lymphomas, leukemias, prostate cancer, lung cancer, colorectal cancer, soft tissue cancers, biopsies, skin cancer, brain cancer, liver cancer, ovarian cancer, and pancreatic cancer. Kits containing one or more components, such as the primers or polymerases of the invention, optionally with an identifying description or label or instructions relating to their use in the methods of the present invention are also provided by the present invention.

### BRIEF DESCRIPTION OF THE DRAWINGS

Figure 1 is an illustration of one exemplary embodiment of the invention for linear amplification of total RNA using single stranded RNA molecules as the target polynucleotides. The figure shows an embodiment of a "round one" as disclosed herein by pointing out select aspects of the invention.
Figure 2 is an illustration of another embodiment of the invention to demonstrate strand reversal such that DNA templates that produce "sense" aRNA are converted to produce "antisense" aRNA.
Figure 3 shows the results of amplification using the methods of the invention in comparison to the methods of US Patent Application 10/062,857, filed October 25, 2001 and published as US2003/0022194 on January 30, 2003. See Example 1 herein.
Figure 4 shows the result of quantitative PCR analyses of the products from Figure 3. See Example 2 herein.

### MODES OF PRACTICING THE INVENTION

### A. Definitions

A "polynucleotide" is a polymeric form of nucleotides of any length, either ribonucleotides or deoxyribonucleotides. This term refers only to the primary structure of the molecule. Thus, this term includes double- and single-stranded DNA and RNA. It also includes known types of modifications including labels known in the art, methylation, "caps", substitution of one or more of the naturally occurring nucleotides with an analog, and internucleotide modifications such as uncharged linkages (e.g., phosphorothioates, phosphorodithioates, etc.), appendant moieties (including proteins such as nucleases, toxins, antibodies, signal peptides, poly-L-lysine, etc.), intercalators (e.g., acridine, psoralen, etc.), chelators (e.g., metals, radioactive metals, etc.), alkylators, modified linkages (e.g., alpha anomeric nucleic acids, etc.), as well as unmodified forms of the polynucleotide.

A "target polynucleotide" or "target sequence," as used herein, contains a polynucleotide sequence of interest, for which amplification is desired. The target sequence may be known or not known, in terms of its actual sequence. Generally, a "template," as used herein, is a polynucleotide that contains the target polynucleotide sequence. In some instances, the terms "target sequence," "template DNA," "template RNA," "template polynucleotide," "target nucleic acid," "target polynucleotide," and variations thereof, are used interchangeably.

The primer regions containing known sequences, as used herein, are selected to be "substantially" complementary to each specific sequence to be amplified, i.e.; the primers should be sufficiently complementary to hybridize to their respective targets. Therefore, the primer sequence need not reflect the exact sequence of the target, and can, in fact be "degenerate." Non-complementary bases or longer sequences can be interspersed into the primer, provided that the primer sequence has sufficient complementarity with the sequence of the target to be amplified to permit hybridization and extension.

Primer regions containing random sequences, as used herein, are not necessarily known to be complementary to target sequences to be amplified, but preferably include sequences that are sufficiently complementary to target sequences to permit primer extension reactions to occur via polymerase activity. Primers may be of any length suitable for hybridization and primer extension under the conditions used. As noted above, primers may be random such that they contain heterologous sequences. Primers heterologous in lengths may also be used in the practice of the invention. Primers may be DNA, RNA or a chimeric combination thereof in structure. Preferred random primer lengths are from about four nucleotides to about 10 nucleotides. Even more preferred are random primers of nine or about nine nucleotides in length. Primers of known sequences may be of lengths from about 12 to about 50 to 100 nucleotides in length. The term "amplify" is used in the broad sense to mean creating an amplification product which may include, for example, additional target molecules, or target-like molecules or molecules complementary to the target molecule, which molecules are created by virtue of the presence of the target molecule in the sample. In the situation where the target is a nucleic acid, an amplification product can be made enzymatically with RNA polymerases with the involvement of DNA polymerases in generating double stranded DNA.

"Amplification," as used herein, generally refers to the process of producing multiple copies of a desired sequence. "Multiple copies" mean at least 2 copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The present invention also provides methods for amplifying mRNA. As such, the subject invention provides methods of producing amplified amounts of RNA from a starting target mRNA. By amplified amounts is meant that for each starting mRNA, multiple corresponding amplified RNAs (aRNAs) are produced where the amplified RNA has a sequence identical to or complementary to the initial mRNA. By corresponding is meant that the aRNA shares a substantial amount of sequence identity with the starting mRNA or its complement. Substantial amount means at least 95%, usually at least 98% and more usually at least 99%, and sequence identity is determined using the BLAST algorithm, as described in Altschul et al. (1990), J. Mol. Biol. 215:403-410 (using the published default setting, i.e. parameters w=4, t=17). Generally, the number of corresponding aRNA molecules produced for each starting mRNA during the linear amplification methods will be at least about 10, usually at least about 50 and more usually at least about 100, where the number may be as great as 600 or greater, but often does not exceed about 5000. Fold amplification of mRNAs by the present invention is from about 1000 fold per round to about 5000 fold per round. As used herein, the term "linker" or "linker sequence" refers to a specific nucleic acid sequence that may serve to link primer regions to promoter regions as disclosed herein.

A "microarray" is a linear or two-dimensional array of preferably discrete regions, each having a defined area, formed on the surface of a solid support. The density of the discrete regions on a microarray is determined by the total numbers of target polynucleotides to be detected on the surface of a single solid phase support, preferably at least about 50/cm², more preferably at least about 100/cm², even more preferably at least about 500/cm², and still more preferably at least about 1,000/cm². As used herein, a DNA microarray is an array of oligonucleotide primers or cDNAs placed on a chip or other surfaces. Since the position of each particular group of primers or cDNAs in the array is known, the identities of the target polynucleotides can be determined based on their binding to a particular position in the microarray.

The term "label" refers to a composition capable of producing a detectable signal indicative of the presence of the target polynucleotide in an assay sample. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means.

The terms "support" or "solid medium" refer to conventional supports such as beads, particles, dipsticks, fibers, filters, membranes and silane or silicate supports such as glass slides.

As used herein, a "biological sample" refers to a sample of tissue or fluid isolated from an individual, including but not limited to, for example, blood, plasma, serum, spinal fluid, lymph fluid, the external sections of the skin, respiratory, intestinal, and genitourinary tracts, tears, saliva, milk, cells (including but not limited to blood cells), tumors, organs, and also samples of in vitro cell culture constituents.

The term "biological sources" as used herein refers to the sources from which the target polynucleotides are derived. The source can be any form of "biological sample" as described above, including but not limited to, cell, tissue or fluid. "Different biological sources" can refer to different cells, tissues or organs of the same individual, or cells, tissues or organs from different individuals of the same species, or cells, tissues or organs from different species. The term may also refer to cells, especially human cells, such as those that are malignant or otherwise associated with cancer, especially breast cancer; and cells that are laser-captured (laser capture microdissection) from fixed tissues from model organisms of human diseases or actual human tissue (postmortem or biopsy material).

A "complex" is an assembly of components. A complex may or may not be stable and may be directly or indirectly detected. For example, as is described herein, given certain components of a reaction, and the type of product(s) of the reaction, existence of a complex can be inferred. For purposes of this invention, a complex is generally an intermediate with respect to the final amplification product(s).

A "primer" or "linker" sequence of a polynucleotide or oligonucleotide is a contiguous sequence of 2 or more bases. Such a sequence is at least about any of 3, 5, 10, 15, 20, 25 contiguous nucleotides.

A region, portion, or sequence which is "adjacent" to another sequence directly abuts that region, portion, or sequence. For example, an RNA portion which is adjacent to a 5' DNA portion of a composite primer directly abuts that region.

A "reaction" or "reaction mixture" is an assemblage of components, which, under suitable conditions, react to form a complex (which may be an intermediate) and/or a product(s).

It must be noted that as used in this specification and the appended claims, the singular forms "a", "an" and "the" include corresponding plural references unless the context clearly dictates otherwise.

"Expression" includes transcription of a deoxyribonucleic acid and/or translation of a ribonucleic acid. The term thus relates to the transcription of DNA templates, such as transcription to produce aRNA as disclosed herein.

As used herein, the term "comprising" and its cognates are used in their inclusive sense; that is, equivalent to the term "including" and its corresponding cognates.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, depend upon, for example, the nature of the nucleotide sequence, temperature, and buffer conditions. These conditions also depend on what event is desired, such as hybridization, cleavage, strand extension or transcription.

The term "3"' (three prime) generally refers to a region or position in a polynucleotide or oligonucleotide 3' (downstream) from another region or position in the same polynucleotide or oligonucleotide.

The term "5 "' (five prime) generally refers to a region or position in a polynucleotide or oligonucleotide 5' (upstream) from another region or position in the same polynucleotide or oligonucleotide.

The term "3'-DNA portion," "3'-DNA region," "3'-RNA portion," and "3'-RNA region," refer to the portion or region of a polynucleotide or oligonucleotide located towards the 3' end of the polynucleotide or oligonucleotide, and may or may not include the 3' most nucleotide(s) or moieties attached to the 3' most nucleotide of the same polynucleotide or oligonucleotide. The 3' most nucleotide(s) can be preferably from about 1 to about 20, more preferably from about 3 to about 18, even more preferably from about 5 to about 15 nucleotides.

The term "5'-DNA portion," "5'-DNA region," "5'-RNA portion," and "5'-RNA region," refer to the portion or region of a polynucleotide or oligonucleotide located towards the 5' end of the polynucleotide or oligonucleotide, and may or may not include the 5' most nucleotide(s) or moieties attached to the 5' most nucleotide of the same polynucleotide or oligonucleotide. The 5' most nucleotide(s) can be preferably from about 1 to about 20, more preferably from about 3 to about 18, even more preferably from about 5 to about 15 nucleotides.

"Detection" includes any means of detecting, including direct and indirect detection. For example, "detectably fewer" products may be observed directly or indirectly, and the term indicates any reduction (including no products). Similarly, "detectably more" product means any increase, whether observed directly or indirectly.

Unless defined otherwise all technical and scientific terms used herein have the same meaning as commonly understood to one of ordinary skill in the art to which this invention belongs.

### B. Embodiments shown in the Figures

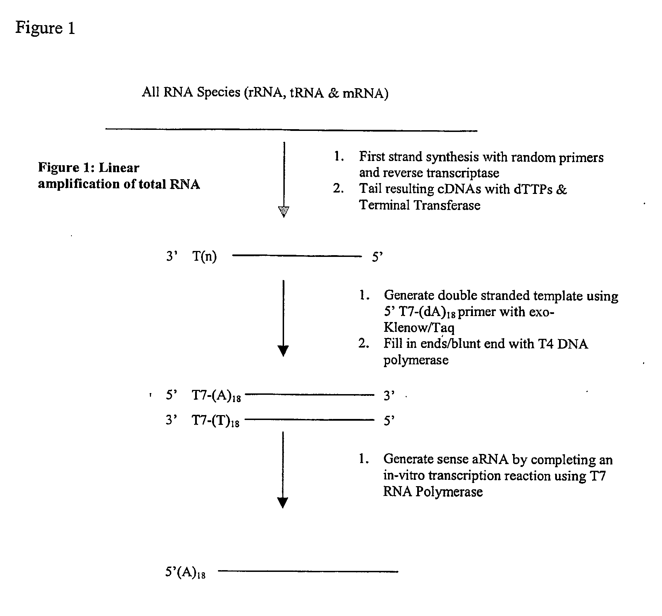

With respect to Figure 1, step 1 of the first arrow produces the first strand DNA via synthesis from a first oligonucleotide that comprises a random primer sequence with reverse transcriptase activity. As described above, the first oligonucleotide may also comprise a defined sequence linked directly or indirectly to the 5' end of the random primer sequence. After synthesis of the first strand DNA, its 3' end is tailed (in this exemplification) with terminal deoxyribonucleotidyl transferase activity to include a poly dT tail (step 2 of the first arrow), although dA, dC, or dG homopolymer tails can be introduced by use of the corresponding dATP, dCTP, or dGTP.

Step 1 of the second arrow refers to the production of the second strand DNA via the use of a second oligonucleotide that comprises a homopolymer primer (complementary to the tail added to the 3' end of the first strand DNA) linked, at the 5' end of the homopolymer primer, to a T7 RNA polymerase promoter sequence. Although not shown in this figure, but as noted above, a linker sequence may be optionally present between the homopolymer primer and the promoter sequence. And while "exo⁻ Klenow/Taq" refers to the use of a combination of exonuclease deficient Klenow and Taq polymerase as the DNA dependent DNA polymerase activity used, other DNA polymerases may also be used.

Step 2 of the second arrow refers to the extension of the 3' end of the first strand DNA to be complementary to the second oligonucleotide used to prime synthesis of the second strand DNA as well as to "finishing" the ends of the resultant double stranded DNA to be blunt ended, exemplified by using T4 DNA polymerase as a DNA polymerase with 3' exonuclease activity.

Step 1 of the third arrow refers to the production of "sense" amplified RNA (aRNA) via an *in vitro* transcription (IVT) reaction using T7 RNA polymerase to initiate transcription from the T7 promoter sequence present in the second oligonucleotide used. If a promoter sequence was linked to the first oligonucleotide used in step 1 of the first arrow, an IVT reaction using an RNA polymerase which uses the promoter sequence as the cognate promoter may be performed to produce "antisense" aRNA. Again, "sense" and "antisense" aRNA is by reference to the original RNA molecule templates, the sequence of which are defined herein as "sense".

Following "round one" as shown in Figure 1, the resultant aRNA may be used in a "round two" amplification. In one embodiment, and where the first oligonucleotide in step 1 of the first arrow did not comprise a defined sequence, "round two" is conducted by completing first strand synthesis via random primers hybridized to the "sense" aRNA produced from "round one" as shown in Figure 1. The second strand DNA is then synthesized with a 5'-T7-oligo(dA) primer followed by an IVT reaction with T7 RNA polymerase. This would complete a second round of amplification. Where the first oligonucleotide in step 1 of the first arrow does include a defined sequence, the "round two" amplification may be performed by using the defined sequence as a primer in place of the random primers.

In embodiments of the invention where oligo dT primers were used in combination with the first oligonucleotides in step 1 of the first arrow, oligo dT primers may be used in combination with the random primers or the defined sequence primers to synthesize the first strand DNA in "round two".

With respect to Figure 2, it illustrates strand reversal (for the amplified RNA product) via two rounds of linear amplification. Strand reversal enables the aRNA to be synthesized in a labeled form in the second IVT reaction and used as probes in certain applications of the invention. Non-limiting examples of such applications include hybridization to microarrays such as the Affymetrix's GeneChip^{™} and Agilent arrays.

Step 1 of the first arrow refers to optional embodiments of the invention wherein particular populations of RNA molecules are enriched prior to amplification. The enrichment for polyadenylated RNA molecules (denoted by the "Aₙ" after the first arrow) is shown as an exemplification. The enrichment of particular populations may be by positive selection for one or more desired populations or negative selection by removal of one or more undesired populations. For example, and without limiting the invention, the mRNA population may be positively selected by use of the polyadenylated 3' tail of mRNA molecules. This can be performed by standard means known in the art, including those based upon oligo dT mediated isolation of polyadenylated mRNA (such as, but not limited to, use of solid media like beads, column media, or filters coupled to oligo dT sequences that are used to hybridize to polyadenylated mRNA based on basepair complementarity between the polyadenylated tail and the oligo dT sequence). Other RNA species that do not bind the oligo dT medium used are washed away to permit isolation of the polyadenylated RNA for use in the instant invention.

Alternatively, polyadenylated RNA can be enriched by removal of other RNA species, such as the removal of rRNA as a non-limiting example. The removal of rRNA can be by use of standard means known in the art, including those based upon the use of ribosomal RNA sequences that may be coupled to (immobilized on) a solid medium. These ribosomal RNA sequences are complementary to the rRNA molecules to be removed such that the rRNA molecules will hybridize to the ribosomal RNA sequences. As a non-limiting example, the ribosomal RNA sequences are biotinylated such that after hybridization with rRNA molecules (e.g. 18S and 28S rRNAs), the double stranded duplex RNAs may be removed following binding to avidin, such as streptavidin immobiliaed on the surface of beads, particularly magnetic beads, by removal of the beads. Other non-limiting methods include the use of biotinylated DNA oligos complementary to rRNA molecules followed by removal of the DNA-RNA heteroduplexes from the solution.

Of course the above methods can be reversed if desired to use oligo dT mediated means to remove polyadenylated RNA to enrich for rRNA molecules or to use complementary rRNA sequences to enrich for rRNA molecules. In a further alternative embodiment, polyadenylated RNA molecules can be selected by use of an oligo dT primer as the primer sequence in the first oligonucleotide of the invention as described above (followed by synthesis of the first strand DNA and its tailing by terminal transferase activity). The advantages of enrichment include the benefits of a greater "specific activity" in the enriched species. As a non-limiting example, enrichment of mRNAs allows for a greater "specific activity" of mRNA (i.e. greater proportion of mRNA versus other RNA species, such as ribosomal RNA, in total RNA).

Step 1 of the second arrow refers to the production of the first strand DNA via synthesis from a first oligonucleotide, that comprises a random primer sequence (shown as a random nonamer as an exemplification) linked to a known (or defined) sequence exemplified as a decamer of dG, with reverse transcriptase activity. Step 2 of the second arrow refers to the tailing of the 3' end of the first strand DNA after its synthesis with terminal deoxyribonucleotidyl transferase activity. The exemplification shows the introduction of a poly dT tail although dA, dC, or dG homopolymer tails can be introduced by use of the corresponding dATP, dCTP, or dGTP.

The third arrow refers to the production of the second strand DNA via the use of a second oligonucleotide that comprises a homopolymer primer (complementary to the tail added to the 3' end of the first strand DNA) linked, at the 5' end of the homopolymer primer, to a T7 RNA polymerase promoter sequence. Although not shown in this figure, but as provided by the invention, a linker sequence may be optionally present between the homopolymer primer and the promoter sequence. And while "exo⁻ Klenow/Taq" refers to the use of a combination of exonuclease deficient Klenow and Taq polymerase as the DNA dependent DNA polymerase activity used, other DNA polymerases may also be used.

Although not shown in this exemplification, the third arrow can also refer to the extension of the 3' end of the first strand DNA to be complementary to the second oligonucleotide used to prime synthesis of the second strand DNA (or blunt ending the double stranded DNA by the use of T4 DNA polymerase as a non-limiting example).

The fourth arrow refers to the production of "sense" aRNA via an *in vitro* transcription (IVT) reaction using T7 RNA polymerase to initiate transcription from the T7 promoter sequence present in the second oligonucleotide used. Again, "sense" aRNA is by reference to the original polyadenylated RNA molecule templates, the sequence of which are defined herein as "sense".

The above summarizes the portion of Figure 2 indicated on the left therein as "1^{st}" round (or "round one") amplification. The lower portion of Figure 2 illustrates "round two" (indicated as "2^{nd}" round) wherein a promoter is introduced on the other end of the double stranded DNA molecule to permit the production of "antisense" aRNA via an IVT reaction. This occurs as follows.

Following the "1^{st}" round as shown in Figure 2, the resultant aRNA is used to produce a first strand DNA using an oligonucleotide comprising a primer sequence that is complementary to the known (defined) sequence (dG decamer in this exemplification) and comprising a promoter sequence linked to the 5' end of the known sequence. Step 1 of the fifth arrow in Figure 2 exemplifies the use of an oligonucleotide comprising an 18mer of dG linked at its 5' end to a T7 RNA polymerase promoter, although other primer sequences (depending on the "known" sequence used) and other promoter sequences may be used in the practice of the invention. After synthesis of the first strand DNA with reverse transcriptase activity, step 2 of the fifth arrow refers to the synthesis of the second strand DNA from an oligonucleotide that is complementary (and hybridized) to the tail added in step 2 of the second arrow with "exo⁻ Klenow/Taq", which refers to the use of a combination of exonuclease deficient Klenow and Taq polymerase as the DNA dependent DNA polymerase activity used. Other DNA polymerases may also be used.

This produces a double stranded DNA molecule with a T7 promoter which can initiate transcription to produce "antisense" aRNA in an IVT reaction as indicated by step 1 of the sixth arrow.

As noted above, the exemplification of tailing reactions as introducing homopolymers of dT residues is non-limiting. Similarly, the exemplification of using a dG polymer in first strand DNA synthesis is also non-limiting. The use of other homopolymeric nucleotide sequences will also work as long as base pairing rules are maintained (i.e., A-T and G-C) for hybridization of a complementary sequence.

### C. General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R.I. Freshney, ed., 1987); "Methods in Enzymology" (Academic Press, Inc.); "Current Protocols in Molecular Biology" (F.M. Ausubel et al., eds., 1987, and periodic updates); "PCR: The Polymerase Chain Reaction", (Mullis et al., eds., 1994).

Primers, oligonucleotides and polynucleotides employed in the present invention can be generated using standard techniques known in the art.

While the present invention may be most commonly practiced in solution, all or part thereof may be practiced as part of a solid (or immobilized) or *in situ* state. For example, and without limiting the invention, synthesis of the first cDNA strand may be conducted *in situ* or with a first primer promoter oligonucleotide that is immobilized on a solid support (such as, but not limited to, a bead, or a membrane or the surface of a solid container).

### D. Promoter-Primer oligonucleotides

In some embodiments, the methods employ a promoter sequence for transcription which is provided by a promoter-primer oligonucleotide (or oligonucleotide that comprises both a primer sequence and a promoter sequence). A promoter-primer for use in the methods and compositions of the present invention is a single-stranded polynucleotide, generally DNA, comprising a promoter sequence that is designed for formation of a double stranded promoter of an RNA polymerase, and a portion capable of hybridizing to a template sequence, preferably at or near its 3' end. In one embodiment, the promoter sequence is located in the 5' portion of the oligonucleotide and the hybridizing sequence is located in the 3' portion of the oligonucleotide. In another embodiment, and typically, the promoter and hybridizing sequences are different sequences. In another possible embodiment, the promoter and hybridizing sequences overlap in sequence identity. In yet another embodiment, the promoter and hybridizing sequences are the same sequence, and thus are in the same location on the promoter-primer. In the embodiments wherein hybridization of the promoter-primer to a template results in a duplex comprising an overhang, DNA polymerase may be used to fill in the overhang to create a double stranded promoter capable of effecting transcription by a suitable RNA polymerase.

A number of RNA polymerase promoters may be used for the promoter region of the promoter-primer. Suitable promoter regions will be capable of initiating transcription from an operationally linked DNA sequence in the presence of ribonucleotides and an RNA polymerase under suitable conditions. The promoter region will usually comprise between about 15 and 250 nucleotides, preferably between about 17 and 60 nucleotides, from a naturally occurring RNA polymerase promoter, a consensus promoter region, or an artificial promoter region, as described in Alberts et al. (1989) in Molecular Biology of the Cell, 2d ed. (Garland Publishing, Inc.). In general, prokaryotic promoters are preferred over eukaryotic promoters, and phage or virus promoters are most preferred. As used herein, the term "operably linked" refers to a functional linkage between the affecting sequence (typically a promoter) and the controlled sequence (the mRNA binding site). The promoter sequence can be from a prokaryotic or eukaryotic source.

Representative promoter regions of particular interest include T7, T3 and SP6 as described in Chamberlin and Ryan, The Enzymes (ed. P. Boyer, Academic Press, New York) (1982) pp 87-108. In a preferred embodiment, the RNA polymerase promoter sequence is a T7 RNA polymerase promoter sequence comprising at least nucleotides -17 to +6 of a wild-type T7 RNA polymerase promoter sequence, preferably joined to at least 20, preferably at least 30 nucleotides of upstream flanking sequence, particularly upstream T7 RNA polymerase promoter flanking sequence. Additional downstream flanking sequence, particularly downstream T7 RNA polymerase promoter flanking sequence, e.g. nucleotides +7 to +10, may also be advantageously used. For example, in one particular embodiment, the promoter comprises nucleotides -50 to +10 of a natural class III T7 RNA polymerase promoter sequence.

In some embodiments, the promoter-primer comprises an intervening linker sequence between a promoter sequence and a portion capable of hybridizing to the 3' end of a polynucleotide template. Suitable length of the intervening linker sequence can be empirically determined, and can be at least about 1, 2, 4, 6, 8, 10, 12, 15 or more nucleotides. Suitable sequence identity of the intervening linker sequence can also be empirically determined, and the sequence is designed to preferably, but not necessarily, enhance the degree of amplification and subsequent options as compared to omission of the sequence. In one embodiment, the intervening sequence is a sequence that is designed to provide for enhanced, or more optimal, transcription by the RNA polymerase used. Generally, the sequence is not related (i.e., it does not substantially hybridize) to the target nucleic acid. More optimal transcription occurs when transcriptional activity of the polymerase from a promoter that is operatively linked to said sequence is greater than from a promoter that is not so linked. The sequence requirements within the actual promoter for optimal transcription are generally known in the art as previously described for various DNA dependent RNA polymerases, such as in U.S. Pat. Nos. 5,766,849 and 5,654,142, and can also be empirically determined.

The length of the portion of the promoter-primer that hybridizes to a target template is preferably from about 5 to about 50 nucleotides, more preferably from about 10 to about 40 nucleotides, even more preferably from about 15 to about 35 nucleotides, and most preferably from about 20 to 30 nucleotides. In some embodiments, the hybridizing portion is at least about any of the following: 3, 5, 10, 15, 20; and less than about any of the following: 30, 40, 50, 60. The complementarity of the hybridizing portion is preferably at least about 25%, more preferably at least about 50%, even more preferably at least about 75%, and most preferably at least about 90% to 100%, to its intended binding sequence on the target nucleic acid. For the amplification of polyadenylated target polynucleotides, the primer portion is preferably poly dT of the lengths described above and below.

Primer and promoter-primer oligonucleotides described above and throughout this specification may be prepared using any suitable method, such as, for example, the known phosphotriester and phosphite triester methods, or automated embodiments thereof. Oligonucleotides of the invention can be synthesized by a number of approaches, e.g. Ozaki et at, Nucleic Acids Research, 20:5205-5214 (1992); Agarwal et at, Nucleic Acids Research, 18:5419-5423 (1990); or the like. The oligonucleotides of the invention may be conveniently synthesized on an automated DNA synthesizer, e.g. an Applied Biosystems, Inc. Foster City, Calif.) model 392 or 394 DNA/RNA Synthesizer, using standard chemistries, such as phosphoramidite chemistry, e.g. disclosed in the following references: Beaucage and Iyer, Tetrahedron, 48:2223-2311 (1992); Molko et al, U.S. Pat. Nos. 4,980,460; Koster et al, U.S. Pat. No. 4,725,677; Caruthers et al, U.S. Pat. Nos. 4,415,732; 4,458,066; and 4,973,679; and the like.

Alternative chemistries, e.g. resulting in non-natural backbone groups, such as phosphorothioate, phosphoramidate, and the like, may also be employed provided that the hybridization efficiencies of the resulting oligonucleotides and/or cleavage efficiency of the exonuclease employed are not adversely affected. Preferably, the oligonucleotide is in the range of 20-100 nucleotides in length. More preferably, the oligonucleotide is in the range of 20-85 nucleotides in length. The precise sequence and length of an oligonucleotide of the invention depends in part on the nature of the target polynucleotide to which it binds. The binding location and length may be varied to achieve appropriate annealing and melting properties for a particular embodiment. Guidance for making design choices can be found in many of the above-cited references describing the "Taqman" type of assays. One method for synthesizing oligonucleotides on a modified solid support is described in U.S. Pat. No. 4,458,066. It is also possible to use a primer that has been isolated from a biological source (such as a restriction endonuclease digest of cloned genomic DNA).

In preferred embodiments of the invention, the molar ratio of primers to template sequences is from about 500:1 to about 8000:1. More preferred are molar ratios of about 1000:1, about 1500:1, about 1600:1, about 1700:1, about 1800:1, about 1900:1, about 2000:1, about 2500:1, about 3000:1, about 3500:1, about 4000:1, about 4500:1, about 5000:1, about 5500:1, about 6000:1, about 6500:1, about 7000:1, and about 7500:1. Most preferred are molar ratios of about 1100:1, about 1200:1, about 1300:1, and about 1400:1. Lower ratios of primer to template are preferred to reduce undesirable effects of excess primers in subsequent reactions. Molar ratio of primer to RNA template may be determined by methods known in the art.

### E. DNA polymerase, ribonuclease and RNA polymerase

The amplification methods of the invention employs the following enzymatic activities: DNA polymerase, optionally ribonuclease such as RNase H, and a DNA dependent RNA polymerase. Preferred embodiments of the invention include the use of an RNase H activity, whether present as part of a DNA polymerase activity or as exogenously supplied. Optionally, the methods of the invention include the use of an exonuclease activity (such as but not limited to exonuclease I from *E. coli*, SI nuclease, mung bean exonuclease, or one or more than one single stranded DNA exonuclease in general) to degrade excess primers (not used to prime first strand cDNA synthesis) as needed. Preferred embodiments of the invention include the use of exonuclease I which may be readily inactivated before proceeding on to subsequent reactions. Other preferred embodiments couple the use of exonuclease to degrade excess first primer with the use of an exonuclease deficient polymerase to synthesize the second cDNA strand while protecting the primers used for second strand synthesis.

DNA polymerases for use in the methods and compositions of the present invention are capable of effecting extension of the composite primer according to the methods of the present invention. Accordingly, a preferred polymerase is one that is capable of extending a nucleic acid primer along a nucleic acid template that is comprised at least predominantly of deoxyribonucleotides. The polymerase should be able to displace a nucleic acid strand from the polynucleotide to which the displaced strand is bound, and, generally, the more strand displacement capability the polymerase exhibits (i.e., compared to other polymerases which do not have as much strand displacement capability) is preferable. Preferably, the DNA polymerase has high affinity for binding at the 3'-end of an oligonucleotide hybridized to a nucleic acid strand.

Also preferred for the practice of the invention is where the DNA polymerase does not possess substantial nicking activity. Preferably, the polymerase has little or no 5'->3' exonuclease activity so as to minimize degradation of primer, termination or primer extension polynucleotides. Generally, this exonuclease activity is dependent on factors such as pH, salt concentration, whether the template is double stranded or single stranded, and so forth, all of which are familiar to one skilled in the art. Mutant DNA polymerases in which the 5'->3' exonuclease activity has been deleted, or in which both 5'>3' and 3'->5 exonuclease activity has been deleted are known in the art and are suitable for the amplification methods described herein. Suitable DNA polymerases for use in the methods and compositions of the present invention may include those disclosed in U.S. Pat. Nos. 5,648,211 and 5,744,312, which include exo⁻ Vent (New England Biolabs), exo⁻ Deep Vent (New England Biolabs), Bst (BioRad), exo⁻ Pfu (Stratagene), Bca (Panvera), sequencing grade Taq (Promega), and thermostable DNA polymerases from *Thermoanaerobacter thermohydrosulfuricus*.

It is preferred that the DNA polymerase displaces primer extension products from the template nucleic acid in at least about 25%, more preferably at least about 50%, even more preferably at least about 75%, and most preferably at least about 90%, of the incidence of contact between the polymerase and the 5' end of the primer extension product. In some embodiments, the use of thermostable DNA polymerases with strand displacement activity is preferred. Such polymerases are known in the art, such as those described in U.S. Pat. No. 5,744,312 (and references cited therein). Preferably, the DNA polymerase has little to no proofreading activity.

While the invention simply requires the use of DNA polymerase activity, the invention is preferably practiced with a combination of polymerase activities wherein the individual polymerases are individually selected from exonuclease deficient Klenow, Taq polymerase, and Sequenase^{™}, optionally in the presence of RNase H, in the synthesis of the second strand of the cDNA molecule corresponding to the target polynucleotide. Most preferred is the use of exonuclease deficient Klenow alone or in combination with Taq polymerase in the presence or absence of RNase H. The combination of exonuclease deficient Klenow and Taq polymerase resulted in the unexpected discovery that this combination resulted in improved cDNA synthesis and hence aRNA production over other polymerases. Methods to test and optimize various polymerase activities and conditions, including the identification of activities and conditions which are not suitable for research or commercial applications, for use in the practice of the present invention are known in the art.

Preferred conditions for the use of the exonuclease deficient Klenow and Taq polymerase combination is to permit the Klenow to function at 37 °C followed by an increase in temperature to permit Taq polymerase to function under reduced Klenow activity conditions. In a preferred embodiment of the invention, the two enzymes are added to a mixture of first strand cDNA and random primers when they have been removed (such as, but not limited to, placement in ice or an ice water bath) from heat treatment at about 95 °C. The mixture may then be placed at room temperature for about 5 to about 10 minutes followed by an increase to 37 °C for about 10 to about 30 minutes followed by about 72 °C for about 5 to about 15 minutes. In preferred embodiments of the invention, the mixture is maintained at or above room temperature after addition of random primers and DNA polymerase activity and until completion of synthesis of the second cDNA strand.

In alternate embodiments of the invention, the times and temperatures may be adjusted relative to the above conditions, but the time period for second strand cDNA synthesis will preferrably not exceed about 3 hours and is preferrably completed in the range of about 1 to about 2 hours. Most preferred is the time period to be less than about one hour or about 30 minutes.

Any reverse transcriptase may be used in the practice of the invention, including, but not limited to, Superscript RTII (optionally RNase H minus), "regular" MMLV-RT (with intrinsic RNaseH activity), AMV RT, or combinations thereof.

The ribonuclease for use in the methods and compositions of the present invention is capable of cleaving ribonucleotides in an RNA/DNA hybrid. Preferably, the ribonuclease cleaves ribonucleotides regardless of the identity and type of nucleotides adjacent to the ribonucleotide to be cleaved. It is preferred that the ribonuclease cleaves independent of sequence identity. Examples of suitable ribonucleases for the methods and compositions of the present invention are well known in the art, including ribonuclease H (RNase H) from *E. coli* or RNaseH associated with retroviral reverse transcriptases.

### F. Additional Rounds of Amplification

Where the total levels of starting RNA target polynucleotide is limiting (e.g., < 20 ng of total RNA or <-400 pg of mRNA in the form of poly (A) RNA), the second and further rounds of amplification of the aRNA may be performed according to methods of the present invention. Such additional rounds may of course be performed more than once, such as, but not limited to, twice, three times, or four times.

The amplification, which will typically be at least about 20-40, typically to 50 to 100 or 250-fold, or 500 to 1000-fold, or 500-2000 fold or more per round of amplification, can be achieved from nanogram quantities or less of total RNA (and thus picograms of starting material of poly(A) RNA), and is economical and simple to perform under standard molecular biology laboratory conditions. It is also easily adaptable into kit form.

In an optional embodiment of the invention, the synthesis of one or both cDNA strands is conducted in the presence of a repetitive polynucleotide such as, but not limited to, polymers of deoxyribo- or ribo- nucleotides of adenosine, cytosine, thymidine, guanine, or inosine. Particularly preferred is the use of poly inosine (poly I), the inclusion of which may be especially advantageous in situations where the amount of the target polynucleotide, especially target RNA, is very low, such as in the picogram range. When higher amounts of target are higher, such as in the nanogram range, there is less added benefit to the inclusion of such polymers.

Use of poly I in the purification of total RNA has been evaluated by Winslow et al. (Nucl. Acids Res. 19(12):3251-3253 1991).

### G. Uses: Detection of Polynucleotide Expression and Related Diagnostic Methods

In specific non-limiting embodiments, the present invention provides methods useful for detecting cancer cells, facilitating diagnosis of cancer and the severity of a cancer (e.g., tumor grade, tumor burden, and the like) in a subject, facilitating a determination of the prognosis of a subject, and assessing the responsiveness of the subject to therapy (e.g., by providing a measure of therapeutic effect through, for example, assessing tumor burden during or following a chemotherapeutic regimen). Detection can be based on detection of a polynucleotide that is differentially expressed in a cell, and/or detection of a polypeptide encoded by a polynucleotide that is differentially expressed in a cell. The detection methods of the invention can be conducted *in vitro* or *in vivo*, on isolated cells, or in whole tissues or a bodily fluid (e.g., blood, plasma, serum, urine, and the like).

The aRNA ofRNAs or mRNAs from live or fixed (from tissue sections via laser capture microdissection or other means of dissection), single or multiple cells can be amplified sufficiently to generate the following: labeled probes for hybridization experiments (e.g., DNA microarrays or macroarrays) thus generating gene expression profiles of various cell types or individual cells (in a manner analogous to that disclosed by Serafini et al., U.S. Pat. no. 6,110,711); cDNA libraries that contain mRNAs from selected cells and subsequently used to generate normalized libraries or various subtractive hybridization methodologies; cDNA that then is used for RT-PCR or quantitative RT-PCR (to subsequently quantitate individual mRNAs); cDNA for subsequent use for various methods that yield differentially expressed genes (i.e., differential display and representational difference analysis (RDA)); and amplified RNA that can be used in various subtraction methodologies, such as subtractive hybridization. The amplification of mRNAs from a single cell is one preferred embodiment of the invention and offers advantages in eliminating the possibility of amplifying heterologous mRNA due to the use of two or more cells. However, the invention may also be practiced with a population of about 100 or less cells, about 100-200 cells, about 200-300 cells, about 300-400 cells, about 400-500 cells, about 500-600 cells, about 600-700 cells, about 700-800 cells, about 800-900 cells or about 900-1000 or more cells.

As noted above, the methods of the present invention may be used to detect expression of a particular gene sequence (including, but not limited to, sequences that differ due to genetic polymorphism) on a cell or population of cells by determining the presence or absence of RNA transcribed from the particular gene sequence in the amplified RNA population. The level of expression of the particular gene may also be determined relative to other RNAs amplified within the overall population of amplified RNAs. The level of expression between different tissues or cell types (or different physiological states of the same tissue or cell type) may also be compared by preparing amplified RNAs from the different tissues or cell types (or the same tissue or cell type under different physiological states, such as, but not limited to, cancer and non-cancer cells) and comparing the species of amplified RNAs produced from the different sources. Different physiological states of the invention include, but are not limited to, different drug (or other active agent) induced states, different behavioral states, different developmental states, different states of stimulation, different states of activation or inhibition, or different states of arousal.

The present methods may also be used to produce amplified RNAs that are used as subtractive hybridization probes or used as the templates for members of a cDNA library (after a second round of amplification and without *in vitro* transcription, for example). The techniques for subtractive hybridization are known in the art, and the use of the present methods provide an advantageous means of producing RNAs for use in subtraction. Similarly, the techniques for preparing a cDNA library are known in the art and the present methods provide an advantageous means of producing the member sequences of a library.

### H. Labeling and Detection of Amplified Molecules

Detecting labeled target polynucleotides can be conducted by standard methods used to detect the labeled sequences. For example, fluorescent labels or radiolabels can be detected directly such as incorporating fluorescent nucleotide dyes into cDNA generation using amplified RNA as template. Other labeling techniques may require that a label such as biotin or digoxigenin be incorporated into the DNA or RNA (during amplification or within cDNA generated from amplified RNA) and detected by an antibody or other binding molecule (e.g. streptavidin) that is either labeled or which can bind a labeled molecule itself. For example, a labeled molecule can be an anti-streptavidin antibody or anti-digoxigenin antibody conjugated to either a fluorescent molecule (e.g. fluorescein isothiocyanate, Texas red and rhodamine), or an enzymatically active molecule. Whatever the label on the newly synthesized molecules, and whether the label is directly in the DNA or conjugated to a molecule that binds the DNA (or binds a molecule that binds the DNA), the labels (e.g. fluorescent, enzymatic, chemiluminescent, or colorimetric) can be detected by a laser scanner or a CCD camera, or X-ray film, depending on the label, or other appropriate means for detecting a particular label.

The amplified target polynucleotide can be detected by using labeled nucleotides (e.g. dNTP-fluorescent label for direct labeling; and dNTP-biotin or dNTP-digoxigenin for indirect labeling) incorporated during amplification or by incorporating it during cDNA synthesis when using amplified RNA as template. For indirectly labeled DNA, the detection is carried out by fluorescence or other enzyme conjugated streptavidin or anti-digoxigenin antibodies. The method employs detection of the polynucleotides by detecting incorporated label in the newly synthesized complements to the polynucleotide targets. For this purpose, any label that can be incorporated into DNA as it is synthesized can be used, e.g. fluoro-dNTP, biotin-dNTP, or digoxigenin-dNTP, as described above and are known in the art. In a differential expression system, amplification products derived from different biological sources can be detected by differentially (e.g., red dye and green dye) labeling the amplified target polynucleotides based on their origins.

In a preferred embodiment, amplified RNA is used as the template for incorporating fluorescent nucleotides during the subsequent probe generation via cDNA synthesis.

For detection, light detectable means are preferred, although other methods of detection may be employed, such as radioactivity, atomic spectrum, and the like. For light detectable means, one may use fluorescence, phosphorescence, absorption, chemiluminescence, or the like. The most convenient will be fluorescence, which may take many forms. One may use individual fluorescers or pairs of fluorescers, particularly where one wishes to have a plurality of emission wavelengths with large Stokes shifts (at least 20 nm). Illustrative fluorescers include fluorescein, rhodamine, Texas red, cyanine dyes, phycoerythrins, thiazole orange and blue, etc.

Depending on the particular intended use of the aRNA, the aRNA may be labeled. One way of labeling which may find use in the subject invention is isotopic labeling, in which one or more of the nucleotides is labeled with a radioactive label, such as ³²S, ³²P, ³H, or the like. Another means of labeling is fluorescent labeling in which a fluorescently tagged nucleotide, e.g. CTP, is incorporated into the aRNA product during transcription. Fluorescent moieties which may be used to tag nucleotides for producing labeled antisense RNA include: fluorescein, the cyanine dyes, such as Cy3, Cy5, Alexa 542, Bodipy 630/650, and the like.

### I. Characterization of nucleic acids

The amplification products obtained by the methods of the invention are particularly amenable to further characterization, in part because the products are single stranded. The amplified products, either DNA or RNA, can analyzed using probe hybridization techniques known in the art, such as Southern and Northern blotting on a solid support such as, but not limited to, nitrocellulose. The amplified products can also be analyzed by contacting them with microarrays comprising oligonucleotide probes or cDNAs. The identity of the probes provides characterization of the sequence identity of the amplified products, and thus by extrapolation the identity of the template nucleic acid present in a sample suspected of containing said template nucleic acid. The above hybridization based techniques may also be used to detect expression from a single gene sequence.

### J. Kits

Also provided are kits for use in the subject invention, where such kits may comprise containers, each with one or more of the various reagents (typically in concentrated form) utilized in the methods, including, for example, buffers, the appropriate nucleotide triphosphates (e.g. dATP, dCTP, dGTP, dTTP, dUTP, ATP, CTP, GTP and UTP), reverse transcriptase, DNA polymerase, RNA polymerase, and one or more sequence-specific primers, degenerate primers, random primers, poly-dT primers and corresponding promoter-primers and tagged-primers of the present invention. A label or indicator describing, or a set of instructions for use of, kit components in an mRNA amplification method of the present invention, will also be typically included, where the instructions may be associated with a package insert and/or the packaging of the kit or the components thereof.

Having now generally described the invention, the same will be more readily understood through reference to the following examples which are provided by way of illustration, and are not intended to be limiting of the present invention, unless specified. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

### EXAMPLES

### Example 1: Comparison of amplification techniques

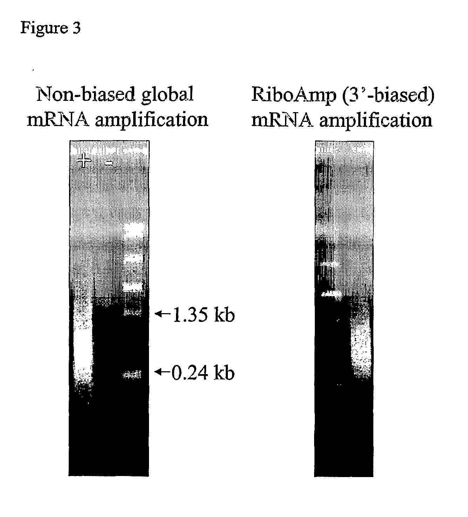

To demonstrate the amplification method and the ability of the method to equally represent the full length of the mRNA, the amplification method outlined in Figure 1 was used with total RNA. One round of the RiboAmp™ amplification process, described in US Patent Application 10/062,857, filed October 25, 2001 and published as US2003/0022194 on January 30, 2003, was performed as a control. The RiboAmp™ process has been observed to have a bias toward amplifying the 3' end of mRNA molecules. 10 ng of total RNA extracted from eukaryotic cells was subjected to either global amplification or RiboAmp amplification. Figure 3 shows gels for each of the amplification methods.

### Example 2: Lack of bias in the global amplification methods of the invention

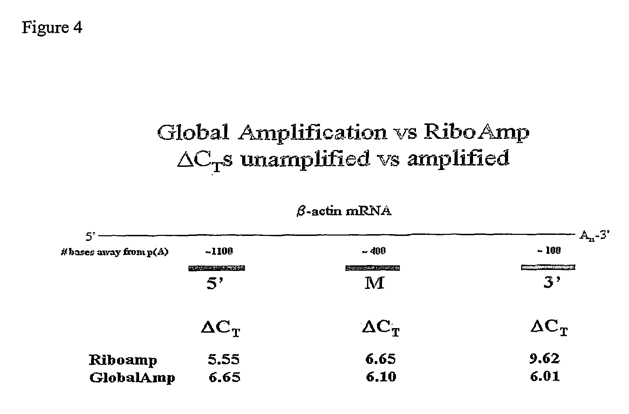

An aliquot of the cDNA synthesis reaction of Example 1 was assayed via Q-PCR using the Light Cycler^{™} to interrogate the presence of three human β-actin amplicons at the 5', middle, and 3' portions of the β-actin mRNA using 3 separate primer sets, each set corresponding to one of the three regions. The results are shown in Figure 4.

The Q-PCR results are expressed as ΔC_{T}, which is the difference between the threshold cycle number (C_{T}) for the sample after IVT amplification and the C_{T} for the sample prior to amplification; the C_{T} being the point at which the fluorescence is determined to be significantly above background via extrapolation of the fluorescence vs. cycle data. The results show that the amplification method of the present invention (indicated as "GlobalAmp") results in an even amount of material derived from each of the 3', middle, and 5' regions of the β-actin mRNA. The ΔC_{T} values for these three regions were 6.65, 6.10, and 6.01, respectively.

This is in comparison to the RiboAmp method, which results in much more material derived from the 3' end of the mRNA (ΔC_{T} values of 5.55, 6.65, and 9.62 for each of the 3', middle, and 5' regions of the beta actin mRNA, respectively). These results demonstrate the non-biased nature of the global amplification method of this invention.

## Claims

1. A method of preparing amplified RNA sequences present in one or more than one target polynucleotide that is single stranded or made single stranded, comprising:
a) forming double stranded cDNA templates containing sequences present in said target polynucleotide, wherein said sequences are operably linked to a promoter, by
i) annealing said single stranded target polynucleotide with a plurality of first oligonucleotides, each comprising a random primer sequence, to form a first complex,
ii) synthesizing a first strand cDNA by reverse transcription of said first complex and adding a homopolymer tail to said first strand cDNA by use of terminal deoxyribonucleotidyl transferase activity,
iii) optionally degrading first oligonucleotides not used in i) or ii) above with exonuclease activity,
iv) annealing said first strand cDNA, after denaturing the mRNA/cDNA hybrid or degrading the RNA from said hybrid, with a second oligonucleotide comprising a primer sequence, complementary to said homopolymer tail and operably linked to a promoter region, to form a population to second complexes, and
v) forming double stranded cDNA templates from said population of second complexes with DNA polymerase activity; and
b) transcribing said cDNA templates with an RNA polymerase capable of initiating transcription via said promoter region to produce amplified RNA (aRNA) containing sequences of said target polynucleotide.

2. The method of claim 1 wherein said DNA polymerase activity is DNA dependent.

3. The method of claim 1 wherein the production of amplified RNA sequences present in one or more than one target polynucleotide is increased by preparing additional double stranded DNA templates, comprising all or part of the sequences of the aRNA, and initiating transcription from the additional templates, said method comprising:
annealing said aRNA to a third oligonucleotide comprising a primer region to form a third complex,
synthesizing the first strand of said additional double stranded DNA templates by reverse transcription of said third complex,
annealing said first strand of additional DNA templates, after denaturing the aRNA/DNA hybrids or degrading the aRNA from said hybrids, with said second oligonucleotide comprising an operably linked promoter region to form a fourth complex,
forming additional double stranded DNA templates from said fourth complex with DNA dependent DNA polymerase activity, and
transcribing said double stranded DNA templates with an RNA polymerase capable of initiating transcription via said promoter region to produce additional amplified RNA (aRNA) containing sequences of said target polynucleotide,
wherein the above annealing, synthesizing, annealing, forming and/or transcribing components of the method are optionally repeated to further amplify said RNA sequences complementary to one or more than one target polynucleotide.

4. A method of amplifying RNA sequences complementary to, or present in, one or more than one target polynucleotide that is single stranded or made single stranded, comprising:
a) forming double stranded cDNA templates containing sequences present in said target polynucleotide, wherein said sequences are operably linked to a promoter region, by
i) annealing said single stranded target polynucleotide with a plurality of first oligonucleotides, each comprising a random primer sequence, to form a first complex,
ii) synthesizing a first strand cDNA by reverse transcription of said first complex and adding a homopolymer tail to said first strand cDNA by use of terminal deoxyribonucleotidyl transferase activity,
iii) optionally degrading first oligonucleotides not used in i) or ii) above with exonuclease activity,
iv) annealing said first strand cDNA, after denaturing the mRNA/cDNA hybrid or degrading the RNA from said hybrid, with a second oligonucleotide comprising a primer sequence, complementary to said homopolymer tail and operably linked to a promoter region, to form a population of second complexes, and
v) forming double stranded cDNA templates from said population of second complexes with DNA dependent DNA polymerase activity; and
b) transcribing said cDNA templates with an RNA polymerase capable of initiating transcription via said promoter region to produce amplified RNA (aRNA) containing sequences complementary of said target polynucleotide;
c) forming additional double stranded DNA templates from said aRNA by
i) annealing said aRNA with a third oligonucleotide comprising a primer sequence operably linked to a promoter region to form a third complex,
ii) synthesizing the first strand of said additional DNA template by reverse transcription of said third complex,
iii) annealing said first strand of additional DNA template, after denaturing the aRNA/DNA hybrid or degrading the aRNA from said hybrid, with said second oligonucleotide to form a population of fourth complexes, and
iv) forming additional double stranded DNA templates from said population of fourth complexes with DNA dependent DNA polymerase activity; and
d) transcribing said additional DNA templates with an RNA polymerase capable of initiating transcription via the promoter region of said third oligonucleotide to produce amplified RNA (aRNA) containing sequences complementary to said target polynucleotide or via the promoter region of said second oligonucleotide to produce aRNA containing sequences of said target polynucleotide.

5. The method of claim 4 wherein said formation of additional double stranded DNA templates from said aRNA further comprises degrading third oligonucleotides not used in c) i) or c) ii) with exonuclease activity before forming additional double stranded DNA templates.

6. The method of any one of claims 3 to 5 wherein said third oligonucleotide comprises a random primer region.

7. The method of claim 6 wherein said random primer region comprises at least about six random nucleotides.

8. The method of claim 7 wherein said random primer region comprises at least about nine random nucleotides.

9. The method of any one of claims 3, 4 or 5 wherein said third oligonucleotide comprises a known primer sequence.

10. The method of claim 9 wherein said known primer sequence is complementary to the 3' region of said aRNA.

11. The method of any one of claims 2 to 10 wherein said DNA dependent DNA polymerase activity comprises exonuclease deficient Klenow and Taq polymerase activities.

12. The method of any one of the preceding claims wherein said target polunucleotide is in an RNA preparation containing mRNA, tRNA, and rRNA.

13. The method of claim 12 wherein said RNA preparation is an FFPE derived sample.

14. The method of claim 13 wherein said RNA preparation is enriched for polyadenylated mRNA molecules.

15. The method of any one of the preceding claims wherein said target polynucleotide is RNA from a breast cancer cell sample.

16. The method of any one of the preceding claims wherein said random primer sequence comprises at least about six random nucleotides.

17. The method of claim 16 wherein said random primer sequence comprises at least about nine random nucleotides.

18. The method of claim 1, 3 or 4 wherein said first oligonucleotide comprises a T7 promoter region.

19. The method of any one of claims 4 or 5 wherein said third oligonucleotide comprises a T3 or SP6 promoter region.

## Patentansprüche

1. Methode zum Herstellen von amplifizierten RNA-Sequenzen, die in einem oder mehr als einem Ziel-Polynukleotid vorhanden sind, das einzelsträngig ist oder einzelsträngig gemacht ist, umfassend:
a) Bilden von doppelsträngigen cDNA-Matrizen, enthaltend Sequenzen, die in dem Ziel-Polynukleotid vorhanden sind, wobei die Sequenzen operativ geknüpft sind an einen Promotor, durch
i) Annealing des einzelsträngigen Ziel-Polynukleotids mit einer Mehrzahl von ersten Oligonukleotiden, jeweils umfassend eine Zufalls-Primersequenz, um einen ersten Komplex zu bilden,
ii) Synthetisieren einer Erststrang-cDNA durch reverse Transkription des ersten Komplexes und Addieren eines Homopolymer-Schwanzes an die Erststrang-cDNA unter Ausnutzung der terminale Desoxyribonukleotidyltransferase-Aktivität,
iii) wahlweise Abbauen der ersten Oligonukleotide, die nicht in obigem i) oder ii) verwendet wurden, mit der Exonuklease-Aktivität,
iv) Annealing der Erststrang-cDNA, nach dem Denaturieren des mRNA/cONA-Hybrids oder Abbauen der RNA aus dem Hybrid, mit einem zweiten Oligonukleotid, umfassend eine Primersequenz, die zu dem Homopolymer-Schwanz komplementär ist und an eine Promotorregion operativ geknüpft ist, um eine Population von zweiten Komplexen zu bilden, und
v) Bilden von doppelsträngigen cDNA-Matrizen aus der Population von zweiten Komplexen mit DNA-Polymerase-Aktivität; und
b) Transkribieren der cDNA-Matrizen mit einer RNA-Polymerase, die zum Initiieren der Transkription über die Promotorregion fähig ist, um amplifizierte RNA (aRNA) -enthaltende Sequenzen des Ziel-Polynukleotids zu erzeugen.

2. Methode nach Anspruch 1, wobei die DNA-Polymerase-Aktivität DNA-abhängig ist.

3. Methode nach Anspruch 1, wobei die Produktion von amplifizierten RNA-Sequenzen, die in einem oder mehr als einem Ziel-Polynukleotid vorhanden sind, durch Herstellen von zusätzlichen doppelsträngigen DNA-Matrizen erhöht wird, umfassend alle oder einen Teil der Sequenzen der aRNA, und Initiieren der Transkription von den zusätzlichen Matrizen, welcher Methode umfasst:
Annealing der aRNA mit einem dritten Oligonukleotid, umfassend eine Primerregion, um einen dritten Komplex zu bilden,
Synthetisieren des Erststrangs der zusätzlichen doppelsträngigen DNA-Matrizen durch reverse Transkription des dritten Komplexes,
Annealing des Erststrangs der zusätzlichen DNA-Matrizen, nach dem Denaturieren des aRNA/DNA-Hybrids oder Abbauen der aRNA aus den Hybriden, mit dem zweiten Oligonukleotid, umfassend eine operativ verknüpfte Promotorregion, um einen vierten Komplex zu bilden,
Bilden von zusätzlichen doppelsträngigen DNA-Matrizen aus dem vierten Komplex mit DNA-abhängiger DNA-Polymerase-Aktivität, und
Transkribieren der doppelsträngigen DNA-Matrizen mit einer RNA-Polymerase, die zum initiieren der Transkription über die Promotorregion fähig ist, um zusätzliche amplifizierte RNA (aRNA) -enthaltende Sequenzen des Ziel-Polynukleotids zu erzeugen,
wobei das obige Annealing, Synthetisieren, Annealing, Bilden und/oder Transkribieren von Komponenten der Methode optional wiederholt wird, um die RNA-Sequenzen, die zu dem einen oder mehr als einen Ziel-Polynukleotid komplementär sind, weiter zu amplifizieren.

4. Methode zum Amplifizieren von RNA-Sequenzen, die komplementär sind zu, oder vorhanden sind in, einem oder mehr als einem Ziel-Polynukleotid, das einzelsträngig ist oder einzelsträngig gemacht ist, umfassend:
a) Bilden von doppelsträngigen cDNA-Matrizen, enthaltend Sequenzen, die in dem Ziel-Polynukleotid vorhanden sind, wobei die Sequenzen operativ geknüpft sind an eine Promotorregion, durch
i) Annealing des einzelsträngigen Ziel-Polynukleotids mit einer Mehrzahl von ersten Oligonukleotiden, jeweils umfassend eine Zufalls-Primersequenz, um einen ersten Komplex zu bilden,
ii) Synthetisieren einer Erststrang-cDNA durch reverse Transkription des ersten Komplexes und Addieren eines Homopolymer-Schwanzes an die Erststrang-cDNA unter Ausnutzung der terminale Desoxyribonukleotidyltransferase-Aktivität,
iii) wahlweise Abbauen der ersten Oligonukleotide, die nicht in obigem i) oder ii) verwendet wurden, mit der Exonuklease-Aktivität,
iv) Annealing der Erststrang-cDNA, nach dem Denaturieren des mRNA/cDNA-Hybrids oder Abbauen der RNA aus dem Hybrid, mit einem zweiten Oligonukleotid, umfassend eine Primersequenz, die zu dem Homopolymer-Schwanz komplementär ist und an eine Promotorregion operativ geknüpft ist, um eine Population von zweiten Komplexen zu bilden, und
v) Bilden von doppelsträngigen cDNA-Matrizen aus der Population von zweiten Komplexen mit DNA-abhängiger DNA-Polymerase-Aktivität; und
b) Transkribieren der cDNA-Matrizen mit einer RNA-Polymerase, die zum Initiieren der Transkription über die Promotorregion fähig ist, um amplifizierte RNA (aRNA) -enthaltende Sequenzen des Ziel-Polynukleotids zu erzeugen;
c) Bilden von zusätzlichen doppelsträngigen DNA-Matrizen aus der aRNA durch
i) Annealing der aRNA mit einem dritten Oligonukleotid, ufmassend eine Primersequenze, die an eine Promotorregion operativ geknüpft ist, um einen dritten Komplex zu bilden,
ii) Synthetisieren des Erststrangs der zusätzlichen DNA-Matrize, nach dem Denaturieren des aRNA/DNA-Hybrids oder Abbauen der aRNA aus dem Hybrid, mit dem zweiten Oligonukleotid, um eine Population von vierten Komplexen zu bilden, und
iv) Bilden von zusätzlichen doppelsträngigen DNA-Matrizen aus der Population von vierten Komplexen mit DNA-abhängiger DNA-Polymerase-Aktivität; und
d) Transkribieren der zusätzlichen DNA-Matrizen mit einer RNA-Polymerase, die zum Initiieren der Transkription über die Promotorregion des dritten Oligonukleotids fähig ist, um amplifizierte RNA (aRNA) -enthaltende Sequenzen zu erzeugen, die zu dem Ziel-Polynukleotid komplementär sind, oder über die Promotorregion des zweiten Oligonukleotids, um aRNAenthaltende Sequenzen des Ziel-Polynukleotids zu erzeugen.

5. Methode nach Anspruch 4, wobei die Bildung der zusätzlichen doppelsträngigen DNA-Matrizen von der aRNA außerdem das Abbauen von dritten Oligonukleotiden, die in c) i) oder c) ii) nicht verwendet wurden, mit Exonuklease-Aktivität umfasst, bevor die zusätzlichen doppelsträngigen DNA-Matrizen gebildet werden.

6. Methode nach einem der Ansprüche 3 bis 5, wobei das dritte Oligonukleotid eine Zufalls-Primerregion umfasst.

7. Methode nach Anspruch 6, wobei die Zufalls-Primerregion wenigstens etwa sechs willkürliche Nukleotide umfasst.

8. Methode nach Anspruch 7, wobei die Zufalls-Primerregion wenigstens etwa neun willkürliche Nukleotide umfasst.

9. Methode nach einem der Ansprüche 3, 4 oder 5, wobei das dritte Oligonukleotid eine bekannte Primersequenz umfasst.

10. Methode nach Anspruch 9, wobei die bekannte Primersequenz zu der 3'-Region der aRNA komplementär ist.

11. Methode nach einem der Ansprüche 2 bis 10, wobei die DNA-abhängige DNA-Polymerase-Aktivität Exonuklease-defiziente Klenow- und Taq-Polymerase-Aktivitäten umfasst.

12. Methode nach einem der vorangehenden Ansprüche, wobei das Ziel-Polynukleotid in einer RNA-Präparation, enthaltend mRNA, tRNA und rRNA, vorliegt.

13. Methode nach Anspruch 12, wobei die RNA-Präparation eine von FFPE abgeleitete Probe ist.

14. Methode nach Anspruch 13, wobein die RNA-Präparation mit polyadenylierten mRNA-Molekülen angereichert ist.

15. Methode nach einem der vorangehenden Ansprüche, wobei das Ziel-Polynukleotid RNA von einer Brustkrebs-Zellprobe ist.

16. Methode nach einem der vorangehenden Ansprüche, wobei die Zufalls-Primersequenz wenigstens etwa sechs willkürliche Nukleotide umfasst.

17. Methode nach Anspruch 16, wobei die Zufalls-Primersequenz wenigstens etwa neun willkürliche Nukleotide umfasst.

18. Methode nach Anspruch 1, 3 oder 4, wobei das erste Oligonukleotid eine T7-Promotorregion umfasst.

19. Methode nach einem der Ansprüche 4 oder 5, wobei das dritte Oligonukleotid eine T3- oder SP6-Promotorregion umfasst.

## Revendications

1. Procédé pour préparer des séquences d'ARN amplifiées présentes dans un ou plusieurs polynucléotides cible qui sont monocaténaires ou rendus monocaténaires, comprenant les étapes consistant à :
a) former des matrices d'ADNc bicaténaire contenant des séquences présentes dans ledit polynucléotide cible, lesdites séquences étant liées de manière opérationnelle à un promoteur, en
i) annelant ledit polynucléotide cible monocaténaire avec une pluralité de premiers oligonucléotides, chacun comprenant une séquence d'amorce aléatoire, pour former un premier complexe,
ii) synthétisant un premier brin d'ADNc par transcription inverse dudit premier complexe et par ajout d'une queue homopolymère audit premier brin d'ADNc en utilisant une activité désoxyribonucléotidyle transférase terminale,
iii) dégradant facultativement les premiers oligonucléotides non utilisés en i) ou ii) ci-dessus avec une activité exonucléase,
iv) annelant ledit premier brin d'ADNc, après dénaturation de l'hybride ARNm/ADNc ou dégradation de l'ARN à partir dudit hybride, avec un deuxième oligonucléotide comprenant une séquence d'amorce, complémentaire de ladite queue homopolymère et liée de manière opérationnelle à une région promotrice, pour former une population de deuxièmes complexes, et
v) formant des matrices d'ADNc bicaténaire à partir de ladite population de deuxièmes complexes avec une activité ADN polymérase ; et à
b) transcrire lesdites matrices d'ADNc avec une ARN polymérase capable d'initier la transcription via ladite région promotrice pour produire de l'ARN amplifié (ARNa) contenant des séquences dudit polynucléotide cible.

2. Procédé selon la revendication 1 dans lequel ladite activité ADN polymérase est dépendante de l'ADN.

3. Procédé selon la revendication 1 dans lequel la production de séquences d'ARN amplifiées présentes dans un ou plusieurs polynucléotides cibles est augmentée par la préparation de matrices d'ADN bicaténaire supplémentaires, comprenant tout ou partie des séquences de l'ARNa, et l'initiation de la transcription à partir des matrices supplémentaires, ledit procédé comprenant les étapes consistant à :
anneler ledit ARNa à un troisième oligonucléotide comprenant une région d'amorce pour former un troisième complexe,
synthétiser le premier brin desdites matrices d'ADN bicaténaire supplémentaires par transcription inverse dudit troisième complexe,
anneler ledit premier brin des matrices d'ADN supplémentaires, après dénaturation des hybrides ARNa/ADN ou dégradation de l'ARNa à partir desdits hybrides, avec ledit deuxième oligonucléotide comprenant une région promotrice liée de manière opérationnelle pour former un quatrième complexe,
former des matrices d'ADN bicaténaire supplémentaires à partir dudit quatrième complexe avec une activité de polymérase de l'ADN dépendante de l'ADN, et
transcrire lesdites matrices d'ADN bicaténaire avec une ARN polymérase capable d'initier la transcription via ladite région promotrice pour produire de l'ARN amplifié supplémentaire (ARNa) contenant des séquences dudit polynucléotide cible,
dans lequel les étapes du procédé ci-dessus, consistant à anneler, synthétiser, anneler, former et/ou transcrire, sont facultativement répétées pour amplifier encore lesdites séquences d'ARN complémentaires d'un ou plusieurs polynucléotides cible.

4. Procédé pour amplifier des séquences d'ARN complémentaires de, ou présentes dans, un ou plusieurs polynucléotides cible qui sont monocaténaires ou rendus monocaténaires, comprenant les étapes consistant à :
a) former des matrices d'ADNc bicaténaire contenant des séquences présentes dans ledit polynucléotide cible, lesdites séquences étant liées de manière opérationnelle à une région promotrice, en
i) annelant ledit polynucléotide cible monocaténaire avec une pluralité de premiers oligonucléotides, chacun comprenant une séquence d'amorce aléatoire, pour former un premier complexe,
ii) synthétisant un premier brin d'ADNc par transcription inverse dudit premier complexe et par ajout d'une queue homopolymère audit premier brin d'ADNc en utilisant une activité désoxyribonucléotidyle transférase terminale,
iii) dégradant facultativement les premiers oligonucléotides non utilisés en i) ou ii) ci-dessus avec une activité exonucléase,
iv) annelant ledit premier brin d'ADNc, après dénaturation de l'hybride ARNm/ADNc ou dégradation de l'ARN à partir dudit hybride, avec un deuxième oligonucléotide comprenant une séquence d'amorce, complémentaire de ladite queue homopolymère et liée de manière opérationnelle à une région promotrice, pour former une population de deuxièmes complexes, et
v) formant des matrices d'ADNc bicaténaire à partir de ladite population de deuxièmes complexes avec une activité ADN polymérase ADN dépendante ; et
b) transcrire lesdites matrices d'ADNc avec une ARN polymérase capable d'initier la transcription via ladite région promotrice pour produire de l'ARN amplifié (ARNa) contenant des séquences complémentaires dudit polynucléotide cible ;
c) former des matrices d'ADN bicaténaire supplémentaires à partir de l'ARNa en
i) annelant ledit ARNa avec un troisième oligonucléotide comprenant une séquence d'amorce liée de manière opérationnelle à une région promotrice pour former un troisième complexe,
ii) synthétisant le premier brin de ladite matrice d'ADN supplémentaire par transcription inverse dudit troisième complexe ;
iii) annelant ledit premier brin de matrice d'ADN supplémentaire après dénaturation de l'hybride ARNa/ADN ou dégradation de l'ARNa à partir dudit hybride, avec ledit deuxième oligonucléotide pour former une population de quatrièmes complexes, et
iv) formant des matrices d'ADN bicaténaire supplémentaires à partir de ladite population de quatrièmes complexes avec une activité ADN polymérase ADN dépendante ; et
d) transcrire lesdites matrices d'ADN supplémentaires avec une ARN polymérase capable d'initier la transcription via la région promotrice dudit troisième oligonucléotide pour produire de l'ARN amplifié (ARNa) contenant des séquences complémentaires dudit polynucléotide cible ou via la région promotrice dudit deuxième oligonucléotide pour produire de l'ARNa contenant des séquences dudit polynucléotide cible.

5. Procédé selon la revendication 4 dans lequel ladite formation de matrices d'ADN bicaténaire supplémentaires à partir dudit ARNa comprend en outre la dégradation des troisièmes oligonucléotides non utilisés en c) i) ou c) ii) avec une activité exonucléase avant la formation de matrices d'ADN bicaténaire supplémentaires.

6. Procédé selon l'une quelconque des revendications 3 à 5 dans lequel ledit troisième oligonucléotide comprend une région d'amorce aléatoire.

7. Procédé selon la revendication 6 dans lequel ladite région d'amorce aléatoire comprend au moins environ six nucléotides aléatoires.

8. Procédé selon la revendication 7 dans lequel ladite région d'amorce aléatoire comprend au moins neuf nucléotides aléatoires.

9. Procédé selon l'une quelconque des revendications 3, 4 ou 5 dans lequel ledit troisième oligonucléotide comprend une séquence d'amorce connue.

10. Procédé selon la revendication 9 dans lequel ladite séquence d'amorce connue est complémentaire de la région 3' dudit ARNa.

11. Procédé selon l'une quelconque des revendications 2 à 10 dans lequel ladite activité ADN polymérase ADN dépendante comprend des activités Klenow et Taq polymérase déficiente en exonucléase.

12. Procédé selon l'une quelconque des revendications précédentes dans lequel ledit polynucléotide cible est une préparation d'ARN contenant de l'ARNm, de l'ARNt et de l'ARNr.

13. Procédé selon la revendication 12 dans lequel ladite préparation d'ARN est un échantillon dérivé de FFPE.

14. Procédé selon la revendication 13 dans lequel ladite préparation d'ARN est enrichie en molécules d'ARNm polyadénylé.

15. Procédé selon l'une quelconque des revendications précédentes dans lequel ledit polynucléotide cible est de l'ARN issu d'un échantillon de cellules de cancer du sein.

16. Procédé selon l'une quelconque des revendications précédentes dans lequel ladite séquence d'amorce aléatoire comprend au moins environ six nucléotides aléatoires.

17. Procédé selon la revendication 16 dans lequel ladite séquence d'amorce aléatoire comprend au moins environ neuf nucléotides aléatoires.

18. Procédé selon la revendication 1, 3 ou 4 dans lequel ledit premier oligonucléotide comprend une région promotrice T7.

19. Procédé selon l'une quelconque des revendications 4 ou 5 dans lequel ledit troisième oligonucléotide comprend une région promotrice T3 ou SP6.
